# EUROPÄISCHE PATENTSCHRIFT

(11) **EP 1 171 420 B1**
(45) Veröffentlichungstag und Bekanntmachung des Hinweises auf die Patenterteilung: **20.08.2003**
(21) Anmeldenummer: 00926732.9
(22) Anmeldetag: 08.03.2000
(51) Int. Cl.: C07C 253/00, C07C 255/12

(54) **VERFAHREN ZUR HERSTELLUNG VON ACETONCYANHYDRIN**
METHOD OF PRODUCING ACETONE-CYANHYDRIN
PROCEDE POUR PRODUIRE DE LA CYANHYDRINE D'ACETONE

(30) Priorität: 22.04.1999 DE 19918246
(43) Veröffentlichungstag der Anmeldung: 16.01.2002
(73) Patentinhaber: Degussa AG, 40474 Düsseldorf (DE)
(72) Erfinder: BÖRNER, Walter, D-63579 Freigericht (DE); DEUSSER, Liane, D-64390 Erzhausen (DE); MARQUARDT, Ralph, D-60318 Frankfurt am Main (DE); VANHEERTUM, Rudolf, B-2930 Brasschaat (BE); VIEWEG, Karl-Heinz, D-63579 Freigericht (DE)
(86) Internationale Anmeldenummer: EP0002014
(87) Internationale Veröffentlichungsnummer: WO00064861

(56) Entgegenhaltungen:
- DE-B- 1 257 765
- US-A- 3 700 718

## Beschreibung

Die Erfindung richtet sich auf ein verbessertes Verfahren zur kontinuierlichen Herstellung von Acetoncyanhydrin aus Cyanwasserstoff und Aceton, wobei Cyanwasserstoff in Form eines inerte andere Gase enthaltenden Gasgemischs, insbesondere eines Rohgases aus dem BMA- oder Andrussow-Prozeß, zur Herstellung von Cyanwasserstoff eingesetzt wird.

Acetoncyanhydrin, also α-Hydroxy-Isobutyronitril, ist das wichtigste Ausgangsprodukt für alle Derivate der Methacrylsäure und vor allem deren Ester. Technisch wird Acetoncyanhydrin durch basisch katalysierte Addition von Cyanwasserstoff (Blausäure) an Aceton hergestellt. In neutralem und besonders alkalischem Bereich steht Acetoncyanhydrin im Gleichgewicht mit seinen Ausgangskomponenten. Die kommerziellen Verfahren benutzen überwiegend Flüssigphasenprozesse, die sowohl diskontinuierlich als auch kontinuierlich in Gegenwart von Katalysatoren, wie Natronlauge, Kalilauge, Kaliumcarbonat, Natriumacetat/Essigsäure, Pyridin/Essigsäure sowie Anionenaustauscherharzen bei Temperaturen unterhalb 40 °C durchgeführt werden. Beispielhaft wird auf das Verfahren der Rohm und Haas hingewiesen (siehe Ullmann's Encyclopedia of Industrial Chemistry 5^{th} ed. (1985), Seiten 91-92): In diesem Verfahren werden flüssige Blausäure, Aceton und ein basischer Katalysator kontinuierlich in einen Reaktor eingeführt und anschließend das Reaktionsgemisch nach Stabilisierung mit Schwefelsäure und Filtration des Katalysators durch eine zweistufige Destillation zunächst von nicht umgesetzter Blausäure und Aceton und dann von Wasser befreit. Die Abgase aus der ersten Destillationsstufe werden in den Reaktor rezykliert, reines stabilisiertes Acetoncyanhydrin am Sumpf der zweiten Destillationsstufe entnommen. Nachteilig an diesem Verfahren ist, daß aus einem Cyanwasserstoff enthaltenden Gas, beispielsweise einem Rohgas aus dem BMA- oder Andrussow-Prozeß zur Herstellung von Cyanwasserstoff, Cyanwasserstoff verflüssigt werden muß. Die technischen Einrichtungen zur Verflüssigung gasförmiger Blausäure und zum Lagern der flüssigen Blausäure verursachen hohe Investitionskosten und hohe variable Verflüssigungskosten, unter anderem durch den Einsatz von Kühlsole. Hinzu kommt, daß ein größeres Lager an flüssiger Blausäure dem Responsible Care-Programm entgegensteht.

Es ist auch bekannt, anstelle von verflüssigter Blausäure ein Cyanwasserstoff und inerte Gase enthaltendes Gasgemisch, beispielsweise Kokereigase, in einem Verfahren zur Herstellung von Acetoncyanhydrin einzusetzen. Im Verfahren der Reinpreussen AG - siehe Ullmann's Encyklopedie der Technischen Chemie, 4. Auflage, Band 7, Seiten 34-35 - werden Cyanwaserstoff enthaltende Kokereigase nach einer Pottaschewäsche kontinuierlich im Gegenstrom mit Aceton, das 10 % Wasser enthält, gewaschen und die Reaktion zu Acetoncyanhydrin in Gegenwart eines basischen Katalysators in zwei hintereinander geschalteten Gaswaschkolonnen durchgeführt; die Aufarbeitung des Acetoncyanhydrin enthaltenden Reaktionsgemischs umfaßt zwei Aceton-Kolonnen und zwei Kolonnen zur Reinigung des Acetoncyanhydrins. Trotz der sehr aufwendigen Anlage läßt es sich nicht vermeiden, daß mit dem aus der zweiten Gaswaschkolonne austretenden Abgas eine große Menge des Acetons und auch ein Teil nicht umgesetzten. Cyanwasserstoffs ausgetragen wird.

Gemäß DE-AS 12 57 765 läßt sich Acetoncyanhydrin aus verdünnten gasförmigen Cyanwasserstoff enthaltenden Gasen der Blausäuresynthese aus Methan und Ammoniak, d. h. aus dem Rohgas des BMA-Prozeßes, dadurch erhalten, daß man das Gas in flüssiges Aceton oder in eine Lösung von Aceton in einem inerten Lösungsmittel, wie Acetoncyanhydrin, bei einem pH-Wert von 8 bis 8,5 einführt, die Umsetzung bei 0 bis 25 °C durchführt und den pH-Wert des Reaktionsgemischs laufend durch Zusatz von Barytlauge aufrecht erhält. Mit den aus dem Reaktor ausgetragenen inerten Gasen werden zwangsläufig auch eine dem Phasengleichgewicht entsprechende Menge Aceton sowie nicht umgesetzte Blausäure ausgetragen - ausweislich der Beispiele dieses Dokuments bricht Cyanwasserstoff in einer Menge von etwa 0,5 bis 7,2 %, bezogen auf die eingesetzte Menge Blausäure, durch. Die in diesem Dokument beschriebenen Ausführungsformen sind demgemäß kaum für eine Übertragung in einen technischen Maßstab geeignet oder machen zumindest eine nachträgliche Aufarbeitung des Abgases zur Rückgewinnung von Aceton und nicht umgesetzter Blausäure erforderlich. Ein weiterer Nachteil besteht darin, daß der pH-Wert mittels Barytlauge eingestellt wird; demgemäß muß die Acetoncyanhydrin enthaltende flüssige Phase nach deren Stabilisierung mit Schwefelsäure vor der destillativen Reinigung über ein Filter zum Abtrennen von Bariumsulfat geleitet werden. Bei der in diesem Dokument beschriebenen Ausführungsform wird durch die mit dem HCN enthaltenden Gasgemisch eingebrachten inerten Gase das Bildungsgleichgewicht von Acetoncyanhydrin ständig in die Richtung der Ausgangsprodukte verschoben, so daß ein Austrag an Aceton und Cyanwasserstoff unvermeidlich ist. Auch wenn dem nach dem Mammutpumpenprinzip betriebenen, mit Acetoncyanhydrin befüllten Umwälzreaktor kontinuierlich Aceton und Cyanwasserstoff enthaltendes Gas zugeführt werden, läßt sich der Austrag an Reaktanden nicht vermeiden, weil im Reaktor ein pH-Wert von 8 bis 8,5 aufrecht erhalten wird.

Aufgabe der vorliegenden Erfindung ist, demgemäß ein verbessertes Verfahren zur Herstellung von Acetoncyanhydrin aufzuzeigen, bei welchem ein Cyanwasserstoff und inerte Gase enthaltendes Gasgemisch, insbesondere ein Rohgas aus dem BMA- oder Andrussow-Prozeß, eingesetzt wird. Gefunden wurde ein Verfahren zur kontinuierlichen Herstellung von Acetoncyanhydrin, umfassend Umsetzung von Cyanwasserstoff mit Aceton in Gegenwart eines basischen Katalysators und Acetoncyanhydrin in einem Gas-Flüssig-Reaktor, welchem ein Cyanwasserstoff und inerte Gase enthaltendes Gasgemisch und Aceton kontinuierlich zugeführt und aus welchem eine Acetoncyanhydrin enthaltende flüssige Phase und eine die inerten Gase enthaltende Gasphase abgeführt werden, und destillative Abtrennung flüchtiger Bestandteile aus der flüssigen Phase, das dadurch gekennzeichnet ist, daß man die aus dem Reaktor abgeführte Gasphase in einem mit einem bei 100 bis 200 °C (Normaldruck) siedenden Lösungsmittel oder mit sauer stabilisiertem reinen, von Aceton und Cyanwasserstoff im wesentlichen freie Acetoncyanhydrin beaufschlagten Gaswäscher von nicht umgesetztem Cyanwasserstoff und Aceton befreit und die erhaltene Waschphase dem Gas-Flüssig-Reaktor zuführt. Die Unteransprüche richten sich auf bevorzugte Ausführungsformen des erfindungsgemäßen Verfahrens.

Als Waschflüssigkeit wird die aus dem Gas-Flüssig-Reaktor austretende Gasphase, welche die inerten Gase des eingesetzten Cyanwasserstoff enthaltenden Gasgemischs sowie Aceton und nicht umgesetzte Blausäure enthält, mit einem inerten Lösungsmittel, insbesondere ein mit Wasser Azeotrope bildendes Lösungsmittel mit einem Siedepunkt oberhalb 100 °C (Normaldruck) verwendet. Vorzugsweise weist dieses Lösungsmittel einen Siedepunkt im Bereich von 150 bis 200 °C auf, jedoch sind auch höhere oder tiefere Siedepunkte unter der Voraussetzung möglich, daß sich das Lösungsmittel problemlos einerseits von Aceton und andererseits von Acetoncyanhydrin abtrennen läßt. Unter dem Begriff "inert" wird verstanden, daß das Lösungsmittel unter den Betriebsbedingungen keine Reaktion mit Cyanwasserstoff, Aceton und Acetoncyanhydrin eingeht.

Gemäß einer alternativen Ausführungsform wird anstelle des genannten Lösungsmittels stabilisiertes reines Acetoncyanhydrin als Waschflüssigkeit dem Gaswäscher zugeführt. Es ist ein erfindungswesentliches Merkmal, daß es sich bei dem stabilisierten reinen Acetoncyanhydrin um ein sauer stabilisiertes Produkt handelt, das im wesentlichen frei von Aceton und Cyanwasserstoff ist. Zweckmäßigerweise wird ein Teil des erfindungsgemäß erzeugten Acetoncyanhydrins nach dessen Stabilisierung mit einer Säure, insbesondere Schwefelsäure oder Phosphorsäure, und destillativer Abtrennung von Aceton und nicht umgesetzter Blausäure in den Gaswäscher rezykliert. Die aus dem Wäscher austretende flüssige Phase durchströmt anschließend den Gas-Flüssig-Reaktor. Das aus dem Gaswäscher austretende Abgas ist im wesentlichen frei von Cyanwasserstoff und Aceton.

Die Menge Waschflüssigkeit für den Gaswäscher richtet sich maßgeblich nach dem Durchsatz des Cyanwasserstoff enthaltenden Gases, der Löslichkeit von Cyanwasserstoff in dem Waschmedium sowie der Reaktionstemperatur. Bei dem bevorzugten Einsatz von stabilisiertem reinen Acetoncyanhydrin als Waschflüssigkeit wird pro Mol des in den Reaktor eingebrachten Cyanwasserstoffs der Gaswäscher zweckmäßigerweise mit etwa 0,3 bis 3 Mol stabilisiertem reinen Acetoncyanhydrin beaufschlagt. Unter Einsatz eines Cyanwasserstoff enthaltenden Rohgases mit einem HCN-Gehalt von etwa 22 bis 23 Vol.-% (= BMA-Rohgas) hat sich ein Mengenverhältnis von 0,5 bis 2 Mol Acetoncyanhydrin pro Mol Cyanwasserstoff als zweckmäßig erwiesen. Je höher die Einspeisrate an Cyanwasserstoff, desto höher wird auch die zu verwendende Menge Waschflüssigkeit sein.

Im erfindungsgemäßen Verfahren wird vorzugsweise ein BMA-Rohgas oder ein Andrussow-Rohgas eingesetzt. Das aus den genannten üblichen Verfahren zur Herstellung von Cyanwasserstoff resultierende Gasgemisch kann als solches oder nach einer Säurewäsche verwendet werden. Das Rohgas aus dem BMA-Prozeß, bei welchem aus Methan und Ammoniak im wesentlichen Blausäure und Wasserstoff gebildet werden, enthält gemäß Ullmanns's Encyclopedia of Technical Chemistry, 5^{th} ed. (1987), Vol. A8, S. 161-163, typischerweise 22,9 Vol.-% HCN, 71,8 Vol.-% H₂, 2,5 Vol.-% NH₃, 1,1 Vol.-% N₂ und 1,7 Vol.-% CH₄. Im bekannten Andrussow-Prozeß werden aus Methan, Ammoniak und Luftsauerstoff Blausäure und Wasser gebildet. Das Rohgas des Andrussow-Prozesses enthält bei Einsatz von Luftsauerstoff als Sauerstoffquelle gemäß dem zuvor zitierten Dokument typischerweise 8 Vol.-% HCN, 22 Vol.-% H₂, 46,5 Vol. -% N₂, 15 Vol.-% H₂O, 5 Vol.-% CO, 2,5 Vol.-% NH₃ und je 0,5 Vol.-% CH₄ und CO₂.

Beim Einsatz eines nicht säuregewaschenen Rohgases aus dem BMA- oder Andrussow-Prozeß wirkt der im Rohgas enthaltene Ammoniak als Katalysator für das erfindungsgemäße Verfahren. Der im Rohgas enthaltene Ammoniak übersteigt häufig die als Katalysator erforderliche Menge und führt demgemäß gegebenenfalls zu einem zu hohen Gehalt an Ammoniumsulfat in dem mit Schwefelsäure stabilisierten Acetoncyanhydrin. In derartigen Fällen ist es vorteilhaft, das Rohgas zunächst einer Säurewäsche zu unterziehen, um Ammoniak aus dem Rohgas zu eliminieren. Beim Einsatz eines säuregewaschenen Rohgases muß dann allerdings ein geeigneter basischer Katalysator dem Gas-Flüssig-Reaktor in katalytisch wirksamer Menge zugesetzt werden. Im Prinzip können die bekannten anorganischen und organischen basischen Verbindungen als Katalysator eingesetzt werden, bevorzugt werden jedoch organische Amine eingesetzt. Gemäß einer besonders bevorzugten Ausführungsform verwendet man ein tertiäres Amin, insbesondere ein tertiäres Amin mit einem Siedepunkt von mindestens 60 °C. Tertiäre Amine, wie Triethylamin, Tri-n-propylamin, Tri-n-butylamin und N-Methyl-morpholyn eignen sich gut. Zweckmäßigerweise liegt die Katalysator-Einsatzmenge im Bereich von 1 bis 100, insbesondere 1 bis 20 Millimol pro Mol eingesetztem Cyanwasserstoff. Vorzugsweise wird in diesen Ausführungsformen der aminische Katalysator zusammen mit dem Aceton in den Gas-Flüssig-Reaktor eingebracht.

Bei dem für die Acetoncyanhydrinbildung eingesetzten Gas-Flüssig-Reaktor kann es sich um einen Reaktor beliebiger Bauart handeln, sofern ein ausreichender Kontakt zwischen der Gasphase und der Flüssigphase gewährleistet wird. Vorzugsweise handelt es sich bei diesen Reaktoren um mehrstufige Reaktoren. Beispiele geeigneter Reaktoren sind mehrere hintereinander geschaltete Rührkessel, eine Kombination aus Rührkessel und Reaktionsrohr, Reaktoren mit kontinuierlicher Umwälzung (Mammutpumpenprinzip), Blasensäulen und Reaktionskolonnen mit Böden und/oder Packungen. Für die erfindungsgemäße Umsetzung ist eine Reaktionskolonne mit mindestens zwei Trennstufen besonders bevorzugt. Im Falle von Bodenkolonnen können die Böden als Glockenböden oder Siebböden oder in anderer Form ausgebildet sein. Im Falle von Kolonnen mit Packungen werden solche Packungselemente bevorzugt, welche einerseits einen intensiven Gas-Flüssigkeitskontakt ermöglichen andererseits aber den Druckverlust in akzeptalen Grenzen halten. Die optimale Trennstufenzahl wird der Fachmann durch orientierende Versuche und/oder Berechnungen unter Berücksichtigung der Phasengleichgewichte ermitteln.

Zur Wäsche der aus dem Reaktor austretenden Gasphase eignen sich an sich bekannte Gaswäscher mit einer oder vorzugsweise mehreren Trennstufen. Vorzugsweise handelt es sich bei den Gaswäschern um Waschkolonnen, welche frei von Einbauten sein können oder übliche Kolonnenböden enthalten, oder in einem oder in mehreren Kolonnenschüssen mit üblichen Packungselementen befüllt sein können.

Gemäß einer besonders bevorzugten Ausführungsform sind sowohl der Reaktor als auch der Gaswäscher in Form einer mehrstufigen Kolonne ausgebildet. Zweckmäßigerweise wird in einer derartigen Kombination das HCN enthaltende Gasgemisch im unteren Teil der Reaktionskolonne zugeführt und unterhalb dieser Zuführstelle das Acetoncyanhydrin enthaltende Reaktionsgemisch abgeführt. Aceton und, soweit erforderlich, ein basischer Katalysator werden im oberen Bereich der Reaktionskolonne, vorzugsweise im Kopf derselben in diese eingespeist. Vorzugsweise wird das am Sumpf der Reaktionskolonne abgezogene Roh-Acetoncyanhydrin nach der Stabilisierung mit einer Mineralsäure, wie Schwefelsäure, destillativ von Aceton und Blausäure befreit. Das so zurückgewonnene Aceton und die nicht umgesetzte Blausäure werden in die Reaktionskolonne rezykliert. Das aus dem Reaktor austretende Gasgemisch wird in die Waschkolonne geleitet und im Gegenstrom mit stabilisiertem reinen Acetoncyanhydrin gewaschen. "Stabilisiert" steht für einen sauren pH-Wert des Acetoncyanhydrins, "rein" bedeutet, daß zuvor Aceton und Blausäure weitgehend abdestilliert wurden. Das zum Waschen eingesetzte stabilisierte reine Acetoncyanhydrin kann noch Wasser enthalten.

Anstelle der üblicherweise bevorzugten Gegenstromführung der Gasphase und flüssigen Phase in der Reaktionskolonne und im Wäscher können auch mit Statik-Mixer-Elementen bestückte Reaktoren und Gaswäscher verwendet werden, in welchen die flüssige Phase und Gasphase im Gleichstrom geführt werden. Bei einer derartigen Ausführung wird das Roh-Acetoncyanhydrin am Kopf der Reaktionskolonne abgezogen und das Wasch-Acetoncyanhydrin ebenso wie die aus dem Reaktor austretende Gasphase am Boden der Waschkolonne in diese eingespeist.

Die mit dem Cyanwasserstoff enthaltenden Gasgemisch in das System eingebrachte Menge Ammoniak oder ein anderweitig dem System zugeführter basischer Katalysator müssen im Reaktor in einer solchen Menge anwesend sein, daß trotz der mit dem Wasch-Acetoncyanhydrin eingebrachten Säure (= Stabilisator) im größten Teil des Reaktors der Katalysator in katalytisch wirksamer Menge zugegen ist, die Säure also nicht zur vollständigen Neutralisation des basischen Katalysators ausreicht.

Die Acetoncyanhydrinbildung wird üblicherweise bei Normaldruck oder geringem Überdruck oder Unterdruck durchgeführt. Zweckmäßigerweise wird die Umsetzung bei einer Temperatur unter 60 °C, insbesondere unter 40 °C, durchgeführt. Gemäß der besonders bevorzugten Ausführungsform unter Verwendung einer Reaktionskolonne und einer Waschkolonne erfolgt die Umsetzung bei einer Temperatur im Bereich von 20 bis 40 °C. Der Gaswäscher wird vorzugsweise bei einer Temperatur unterhalb der im Reaktor herrschenden Temperatur betrieben. Demgemäß liegt die Temperatur im Gaswäscher unter 40 °C, vorzugsweise unter 30 °C und insbesondere im Bereich zwischen etwa 5 und 25 °C. Bei Verwendung eines auf etwa 5 °C abgekühlten Wasch-Acetoncyanhydrins (stabilisiertes reines Acetoncyanhydrin) werden gute Waschergebnisse erzielt, so daß das Abgas nur noch Spuren an Aceton und Cyanwasserstoff enthält.

Die Aufarbeitung des aus dem Reaktor abgezogenen Roh-Acetoncyanhydrins (Roh-ACH) erfolgt in dem Fachmann bekannter Weise. Zunächst wird das Roh-ACH durch Zugabe einer Säure stabilisiert, sodann werden die Leichtsieder HCN, Aceton und Wasser ein- oder mehrstufig abdestilliert. Eine zweckmäßige Ausgestaltung zur Aufarbeitung des Roh-ACH folgt aus der EP 0 421 237 B1.

Ein wesentlicher Vorteil des erfindungsgemäßen Verfahrens besteht darin, daß Acetoncyanhydrin in einfacher Weise, mit hoher Selektivität und guter Raum-Zeit-Ausbeute unter Verwendung von Cyanwasserstoff enthaltenden Gasgemischen mit hohem Inertgasanteil erhältlich ist. Ein weiterer Vorteil besteht darin, daß das den Gaswäscher verlassende Abgas nur noch Spuren an Aceton, Cyanwasserstoff und Acetoncyanhydrin enthält. Durch die erfindungsgemäße Gaswäsche wird Aceton zuverlässig aus dem Abgas herausgewaschen und steht im Reaktor für die Cyanhydrinbildung wieder zur Verfügung. Die Kombination aus Reaktor und Gaswäscher erlaubt es in einfacher Weise, die Mengenströme so zu steuern, daß Aceton und Blausäureverluste im wesentlichen vermieden werden. Je niedriger der Cyanwasserstoffgehalt im einzusetzenden Gasgemisch ist, desto höher ist allerdings die pro Mol HCN einzusetzende Menge Wasch-Acetoncyanhydrin, um die Acetonverluste im Abgas auf ein technisch relevantes Maß zu reduzieren - das Molverhältnis Wasch-Acetoncyanhydrin zu Cyanwasserstoff ist bei Einsatz von Andrussow-Rohgas größer als 1, bei Einsatz von BMA-Rohgas können die Werte aber auf unter 1 bis etwa 0,5 abgesenkt werden. Durch das erfindungsgemäße Verfahren erübrigt sich der technische Aufwand, um aus einem HCN enthaltenen Rohgas zunächst flüssige Blausäure zu gewinnen. Gleichzeitig erübrigt sich die Bereitstellung eines Lagertanks für flüssige Blausäure. Das erfindungsgemäße Verfahren stellt einen positiven Beitrag im Sinne des "Responsible Care" dar.

### Beispiel

Aus mit Schwefelsäure gewaschenem Rohgas aus dem BMA-Prozeß und Acton wurde Acetoncyanhydrin hergestellt. Als Katalysator diente Triethylamin. Die zur Umsetzung verwendete Apparatur umfaßt eine Reaktionskolonne und eine Waschkolonne mit jeweils 10 Böden. Die Reaktionstemperatur im Reaktor lag im Bereich von 40 bis 25 °C, jene in der Waschkolonne etwa im Bereich von 25 bis 12 °C. Der Wäscher wurde mit stabilisiertem reinen Acetoncyanhydrin mit einer Temperatur von 5 °C beaufschlagt. Die Zusammensetzung der dem System zugeführten und abgeführten flüssigen Phasen und Gasphasen folgt aus der Tabelle 1.

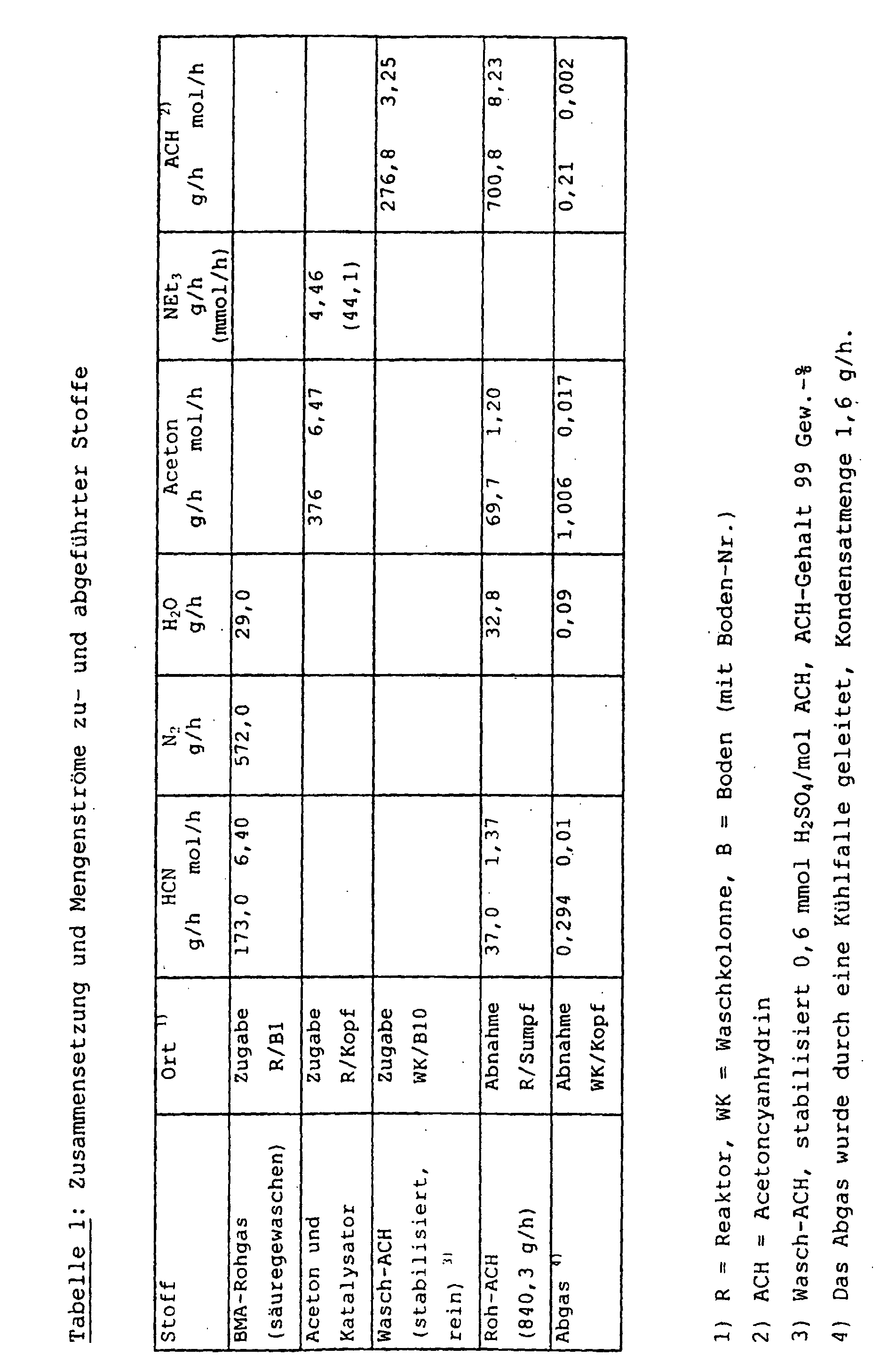

Tabelle 2 zeigt einige Analysendaten der Flüssigphase entlang der Kombination aus Reaktions- und Waschkolonne.

**Tabelle 2:**

| Zusammensetzungen (Angaben in Gew.-%) | | | | |
|---|---|---|---|---|
| | HCN | Aceton | ACH | H₂O |
| Reaktionskolonne | | | | |
| Boden Nr. 2 | 4,65 | 6,3 | 84,5 | 4,6 |
| " Nr. 4 | 2,2 | 12,3 | 83,8 | 1,8 |
| " Nr. 6 | 0,3 | 43,8 | 54,9 | 1,0 |
| " Nr. 8 | 0,1 | 69,3 | 29,9 | 0,7 |
| " Nr. 10 | 0,1 | 77,6 | 21,7 | 0,6 |

| Waschkolonne | | | | |
|---|---|---|---|---|
| Boden Nr. 1 | 0,2 | 59,9 | 39,6 | 0,4 |
| " Nr. 5 | 0,15 | 25,6 | 74,0 | 0,4 |
| " Nr. 8 | 0,2 | 7,0 | 92,3 | 0,5 |

## Patentansprüche

1. Verfahren zur kontinuierlichen Herstellung von Acetoncyanhydrin, umfassend Umsetzung von Cyanwasserstoff mit Aceton in Gegenwart eines basischen Katalysators und Acetoncyanhydrin in einem Gas-Flüssig-Reaktor, welchem ein Cyanwasserstoff und inerte Gase enthaltendes Gasgemisch und Aceton kontinuierlich zugeführt und aus welchem eine Acetoncyanhydrin enthaltende flüssige Phase und eine die inerten Gase enthaltende Gasphase abgeführt werden, und destillative Abtrennung flüchtiger Bestandteile aus der flüssigen Phase,
**dadurch gekennzeichnet,**
**daß** man die aus dem Reaktor abgeführte Gasphase in einem mit einem bei 100 bis 200 °C (Normaldruck) siedenden Lösungsmittel oder mit sauer stabilisiertem reinen, von Aceton und Cyanwasserstoff im wesentlichen freien Acetoncyanhydrin beaufschlagten Gaswäscher von nicht umgesetztem Cyanwasserstoff und Aceton befreit und die erhaltene Waschphase dem Gas-Flüssig-Reaktor zuführt.

2. Verfahren nach Anspruch 1,
**dadurch gekennzeichnet,**
**daß** man die Gasphase mit sauer stabilisiertem reinen, von Aceton und Cyanwasserstoff in wesentlichen freien Acetoncyanhydrin wäscht.

3. Verfahren nach Anspruch 1 oder 2,
**dadurch gekennzeichnet,**
**daß** man als Gas-Flüssig-Reaktor eine mehrstufige Reaktionskolonne verwendet.

4. Verfahren nach Anspruch 1 oder 2,
**dadurch gekennzeichnet,**
**daß** man als Gaswäscher eine mehrstufige Waschkolonne verwendet.

5. Verfahren nach einem der Ansprüche 1 bis 4,
**dadurch gekennzeichnet,**
**daß** das Cyanwasserstoff und inerte Gase enthaltende Gasgemisch eine Zusammensetzung aufweist, wie sie aus dem BMA-Prozeß oder dem Andrussow-Prozeß zur Herstellung von Cyanwasserstoff resultiert.

6. Verfahren nach einem der Ansprüche 1 bis 5,
**dadurch gekennzeichnet,**
**daß** man die Umsetzung in einer mehrstufigen Reaktionskolonne durchführt, indem man in den unteren Teil der Reaktionskolonne das Cyanwasserstoff enthaltende Gasgemisch und in den oberen Teil Aceton einspeist und aus dem Sumpf der Reaktionskolonne die Acetoncyanhydrin enthaltende flüssige Phase abnimmt, diese zwecks Gewinnung von stabilisiertem reinen Acetoncyanhydrin mit einer Säure stabilisiert und dann destillativ von Leichtsiedern befreit, und die an der Reaktionskolonne austretende Gasphase in einer mehrstufigen Waschkolonne im Gegenstrom mit sauer stabilisiertem reinen von Aceton und Cyanwasserstoff in wesentlichen freien Acetoncyanhydrin wäscht.

7. Verfahren nach einem der Ansprüche 1 bis 6,
**dadurch gekennzeichnet,**
**daß** man in den Gas-Flüssig-Reaktor zusätzlich ein organisches Amin als Katalysator kontinuierlich einspeist.

8. Verfahren nach einem der Ansprüche 1 bis 7,
**dadurch gekennzeichnet,**
**daß** man in den Gas-Flüssig-Reaktor kontinuierlich ein säuregewaschenes Rohgas aus dem BMA- oder Andrussow-Prozeß und zusätzlich ein organisches Amin, insbesondere Triethylamin, als Katalysator einspeist.

9. Verfahren nach einem der Ansprüche 1 bis 8,
**dadurch gekennzeichnet,**
**daß** man pro Mol des in den Gas-Flüssig-Reaktor eingeführten Cyanwasserstoffs 0,3 bis 3 Mol sauer stabilisiertes reines, von Aceton und Cyanwasserstoff in wesentlichen freien Acetoncyanhydrin in den Gaswäscher einspeist.

## Claims

1. A process for the continuous production of acetone cyanohydrin, comprising conversion of hydrogen cyanide with acetone in the presence of a basic catalyst and acetone cyanohydrin in a gas/liquid reactor to which acetone and a gas mixture containing hydrogen cyanide and inert gases are supplied continuously and from which a liquid phase containing acetone cyanohydrin and a gas phase containing the inert gases are carried away, and separation of volatile constituents from the liquid phase by distillation,
**characterised in that**
the gas phase which has been carried away out of the reactor is freed of unconverted hydrogen cyanide and acetone in a gas-washer which is subjected to the action of a solvent boiling at 100 to 200 °C (normal pressure) or of acidically stabilised pure acetone cyanohydrin substantially free of acetone and hydrogen cyanide, and the washed phase which is obtained is supplied to the gas/liquid reactor.

2. Process according to Claim 1,
**characterised in that**
the gas phase is washed with acidically stabilised pure acetone cyanohydrin substantially free of acetone and hydrogen cyanide.

3. Process according to Claim 1 or 2,
**characterised in that**
a multi-stage reaction column is used by way of gas/liquid reactor.

4. Process according to Claim 1 or 2,
**characterised in that**
a multi-stage wash column is used by way of gas-washer.

5. Process according to one of Claims 1 to 4,
**characterised in that**
the gas mixture containing hydrogen cyanide and inert gases has a composition such as results from the BMA process or the Andrussow process for the production of hydrogen cyanide.

6. Process according to one of Claims 1 to 5,
**characterised in that**
the conversion is carried out in a multi-stage reaction column by the gas mixture containing hydrogen cyanide being fed into the lower part of the reaction column and by acetone being fed into the upper part and by the liquid phase containing acetone cyanohydrin being removed from the bottom of the reaction column, by said liquid phase being stabilised with an acid for the purpose of obtaining stabilised pure acetone cyanohydrin and then being freed of low-boiling components by distillation, and the gas phase emerging at the reaction column is washed in countercurrent with acidically stabilised pure acetone cyanohydrin substantially free of acetone and hydrogen cyanide in a multi-stage wash column.

7. Process according to one of Claims 1 to 6,
**characterised in that**
an organic amine is additionally fed continuously into the gas/liquid reactor by way of catalyst.

8. Process according to one of Claims 1 to 7,
**characterised in that**
an acid-washed crude gas arising from the BMA process or the Andrussow process and additionally an organic amine, in particular triethylamine, are fed continuously into the gas/liquid reactor by way of catalyst.

9. Process according to one of Claims 1 to 8,
**characterised in that**
0.3 to 3 moles of acidically stabilised pure acetone cyanohydrin substantially free of acetone and hydrogen cyanide are fed into the gas-washer per mole of the hydrogen cyanide introduced into the gas/liquid reactor.

## Revendications

1. Procédé pour la fabrication continue de cyanhydrine d'acétone comprenant la réaction d'acide cyanhydrique avec de l'acétone en présence d'un catalyseur basique et de cyanhydrine d'acétone dans un réacteur gaz-liquide, dans lequel est introduit en continu un mélange gazeux contenant de l'acide cyanhydrique et des gaz inertes, et de l'acétone, et duquel sont retirées une phase liquide contenant de la cyanhydrine d'acétone et une phase gazeuse contenant les gaz inertes, et la séparation par distillation des constituants volatils de la phase liquide,
**caractérisé en ce qu'**
on épure, dans un laveur de gaz alimenté avec un solvant bouillant à 100 à 200°C (pression normale) ou avec une cyanhydrine d'acétone pure, stabilisée par un acide, pratiquement exempte d'acétone et d'acide cyanhydrique, la phase gazeuse retirée du réacteur, de l'acide cyanhydrique et de l'acétone non entrés en réaction, et on envoie la phase de lavage obtenue au réacteur gaz-liquide.

2. Procédé selon la revendication 1,
**caractérisé en ce qu'**
on lave la phase gazeuse avec une cyanhydrine d'acétone pure, stabilisée avec un acide, pratiquement exempte d'acétone et d'acide cyanhydrique.

3. Procédé selon la revendication 1 ou 2,
**caractérisé en ce qu'**
on utilise comme réacteur gaz-liquide une colonne de réaction multi-étagée.

4. Procédé selon la revendication 1 ou 2,
**caractérisé en ce qu'**
on utilise comme laveur de gaz une colonne de lavage multi-étagée.

5. Procédé selon l'une quelconque des revendications 1 à 4,
**caractérisé en ce que**
le mélange gazeux contenant l'acide cyanhydrique et les gaz inertes présente une composition comme celle qui résulte du procédé BMA ou du procédé Andrussow pour la fabrication d'acide cyanhydrique.

6. Procédé selon l'une quelconque des revendications 1 à 5,
**caractérisé en ce qu'**
on effectue la réaction dans une colonne de réaction muti-étagée en introduisant, dans la partie inférieure de la colonne de réaction, le mélange gazeux contenant l'acide cyanhydrique et en introduisant dans la partie supérieure l'acétone, et en soutirant du fond de la colonne de réaction la phase liquide contenant la cyanhydrine d'acétone, on stabilise celle-ci avec un acide pour obtenir une cyanhydrine d'acétone pure stabilisée, puis on l'épure par distillation des substances de bas point d'ébullition, et on lave la phase gazeuse sortant de la colonne de réaction dans une colonne de lavage multi-étagée à contre-courant avec de la cyanhydrine d'acétone pure, stabilisée avec un acide, pratiquement exempte d'acétone et d'acide cyanhydrique.

7. Procédé selon l'une quelconque des revendications 1 à 6,
**caractérisé en ce qu'**
on introduit en plus, en continu, dans le réacteur gaz-liquide, une amine organique comme catalyseur.

8. Procédé selon l'une quelconque des revendications 1 à 7,
**caractérisé en ce qu'**
on introduit en continu, dans le réacteur gaz-liquide, un gaz brut provenant du procédé BMA ou du procédé Andrussow, lavé avec un acide, et, en plus, une amine organique, en particulier la triéthylamine, comme catalyseur.

9. Procédé selon l'une quelconque des revendications 1 à 8,
**caractérisé en ce qu'**
on introduit, par mole d'acide cyanhydrique introduit dans le réacteur gaz-liquide, 0,3 à 3 moles de cyanhydrine d'acétone pure, stabilisée avec un acide, pratiquement exempte d'acétone et d'acide cyanhydrique.
